# EUROPÄISCHE PATENTSCHRIFT

(11) **EP 3 935 044 B1**
(45) Veröffentlichungstag und Bekanntmachung des Hinweises auf die Patenterteilung: **12.04.2023**
(21) Anmeldenummer: 20706334.8
(22) Anmeldetag: 28.02.2020
(51) Int. Cl.: C07D 213/73

(54) **VERFAHREN ZUR HERSTELLUNG VON 4-AMINO-5-METHYLPYRIDON**
PROCESS FOR PREPARING 4-AMINO-5-METHYLPYRIDONE
PROCÉDÉ DE PRODUCTION DE 4-AMINO-5-MÉTHYLPYRIDONE

(30) Priorität: 05.03.2019 EP 19160906
(43) Veröffentlichungstag der Anmeldung: 12.01.2022
(73) Patentinhaber: Bayer Aktiengesellschaft, 51373 Leverkusen (DE); Bayer Pharma Aktiengesellschaft, 13353 Berlin (DE)
(72) Erfinder: PLATZEK, Johannes, 12621 Berlin (DE)
(74) Vertreter: BIP Patents
(86) Internationale Anmeldenummer: PCT/EP2020/055297
(87) Internationale Veröffentlichungsnummer: WO 2020/178177

(56) Entgegenhaltungen:
- CN-B- 103 193 704
- LARS BAERFACKER ET AL: "Discovery of BAY 94-8862: A Nonsteroidal Antagonist of the Mineralocorticoid Receptor for the Treatment of Cardiorenal Diseases", CHEMMEDCHEM, Bd. 7, Nr. 8, 12. August 2012 (2012-08-12) , Seiten 1385-1403, XP055213619, ISSN: 1860-7179, DOI: 10.1002/cmdc.201200081
- NGUYEN CHI HUNG ET AL: "A General Route to 5- and 6-Substituted 4-Amino-2-oxo-1,2-dihydropyridines", SYNTHESIS, GEORG THIEME VERLAG, STUTTGART, DE, Bd. 9, Nr. 39-7881, 1. Januar 1984 (1984-01-01), Seiten 765-766, XP009519718, ISSN: 0039-7881, DOI: 10.1055/S-1984-30963 in der Anmeldung erwähnt

## Beschreibung

Die vorliegende Erfindung betrifft ein neues und verbessertes Verfahren zur Herstellung von 4-Amino-5-methylpyridon der Formel (I)

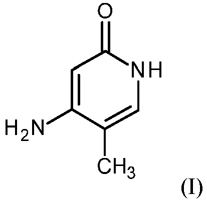

Verbindung der Formel (I) ist ein Schlüssel-Intermediat für die Herstellung von Finerenone (II):

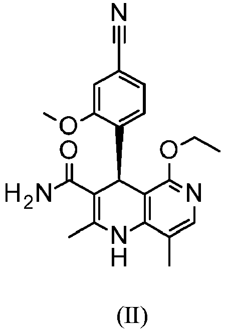

Finerenone (II) wirkt als nicht steroidaler Antagonist des Mineralcorticoid Rezeptors und kann als Mittel zur Prophylaxe und /oder Behandlung von kardiovaskulären und renalen Erkrankungen wie beispielsweise Herzinsuffizienzen und diabetische Nephropathie eingesetzt werden.

Die Verbindung der Formel (II) und deren Herstellungsprozess sind in der WO 2008/104306 A1 und ChemMedChem 2012, 7, 1385 sowie in WO 2016/016287 A1 (Bayer Pharma AG) beschrieben, wobei in beiden Publikationen eine ausführliche Diskussion der Forschungs-Synthese offenbart ist. Nachteilig an der dort beschriebenen Synthese ist die Tatsache, dass sich diese Synthese nicht für ein weiteres großtechnisches Verfahren eignet, da viele Schritte in sehr hoher Verdünnung, mit sehr hohen ReagenzÜberschüssen und damit zu einer relativ geringen Gesamtausbeute verlaufen.

Es bestand daher der Bedarf nach einer großtechnisch praktikablen Synthese, die das Verfahrensintermediat der Formel (I) reproduzierbar in hoher Gesamtausbeute, niedrigen Gestehungskosten und hoher Reinheit liefert und allen regulatorischen Anforderungen entspricht, um die klinischen Prüfungen mit Wirkstoff zu beliefern und für eine spätere behördliche Einreichung verwendet zu werden.

Die Herstellung von Verbindung (I) ist in Synthesis, Seite 765 (1984), beschrieben (Beispiel 3c). Ausgehend vom Hydroxypridon (III), welches in Beispiel 1c der Synthesis-Publikation beschrieben ist wird in siedendem Benzylamin (IV) zur Verbindung (V) umgesetzt. Anschließend wird die Benzylgruppe in Verbindung (V) durch katalytische Hydrierung über Palladium/Kohle ab hydriert. Die Gesamtausbeute über beide Stufen beträgt 62,4 % der Theorie:

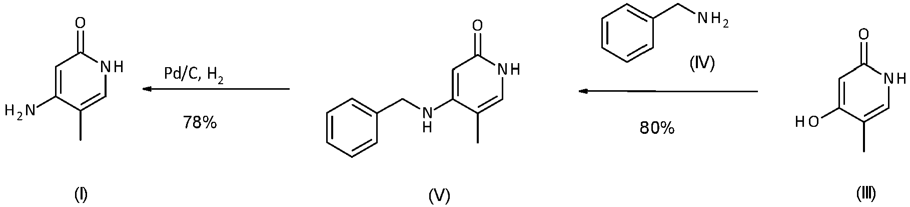

Nachteil des Verfahrens ist die Verwendung von einem sehr großen Überschuss an Benzylamin, für 30 mmol, bezogen auf die Verbindung der Formel (III), werden 30 ml (275,2 mmol) verwendet, das ist ein 9,17-facher Überschuss, bezogen auf Verbindung (III). Das Recycling von überschüssigen Benzylamin ist aufwendig und mit erheblichen Kosten verbunden. Die Reaktion wird in siedendem Benzylamin (185°C) durchgeführt, die Reaktionszeit beträgt 36 Stunden. Solch hohe Temperaturen sind in Standard-Rührwerken nicht durchführbar und erfordern spezielles technisches Equipment. Beim Nachkochen der Vorschrift fiel besonders ein Nebenprodukt (VI) auf, das auf Spuren von Palladium aus der Vorstufe zu (III) zurückzuführen ist:

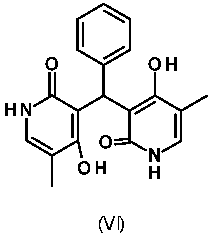

Unter den drastischen Reaktionsbedingungen kommt es zu einer Dehydrierung zum Benzylimin, welches dann zum Benzaldehyd zerfällt (es wird Wasser während der Reaktion gebildet), der Benzaldehyd kondensiert mit Verbindung (III) zu Verbindung (VI). Dieses Nebenprodukt entsteht besonders beim Up-Scalen (bis > 10%) der Ansätze und schleppt sich bis zur Verbindung (I) mit durch. Zur Aufarbeitung werden nach Abkühlung der Reaktionslösung auf Raumtemperatur, die ausgefallenen Kristalle mit Methylethylketon und o-Dichlorbenzol gewaschen und anschließend aus o-Dichlorbenzol umkristallisiert. Auch hier wäre es vorteilhaft auf chlorierte Lösungsmittel zu verzichten und umweltfreundlichere Varianten verfolgen.

Die nachfolgende Debenzylierung läuft in Eisessig, 10 mmol in 200 ml, das sind 2,14 g Verbindung (V) in 200 ml. Dieses entspricht einem 93,45 -fach. Das heißt für 1 kg (V) bräuchte man 93,45 L Essigsäure. Dieses sind riesige Überschüsse, die für ein technisches Verfahren nicht in Frage kommen. Des Weiteren werden für die Umsetzung von 10 mmol (V), 600 mg Pd-Katalysator auf Kohle (10% und 30%) eingesetzt. Das heißt, um 1 kg Verbindung (V) zu debenzylieren, wären 280 g Katalysator notwendig. Auch das ist aus technischer und ökonomischer Sicht nicht sinnvoll. Zur Aufarbeitung wird vom Katalysator abfiltriert und das Filtrat zur Trockene eingedampft, mit Toluol werden Reste Essigsäure herausgeschleppt und der Rückstand in Aceton oder Methylethylketon aufgenommen und abfiltriert. Dieser Vorgang ist beim Upscaling technisch nicht realisierbar, da in Rührwerken nicht zur Trockene eingedampft wird. Außerdem werden drei verschiedene Lösungsmittel für die Isolierung benötigt. Das stark gefärbte Reaktionsprodukt wird anschließend noch per Chromatographie gereinigt (Dichlormethan/MeOH 1:1), auch das möchte man für einen großtechnischen Prozess nach Möglichkeit vermeiden.

Es bestand daher die Aufgabe eine alternative Synthese für das Verfahrensintermediat 4-Amino-5-methylpyridon als Zwischenprodukt für die Herstellung von Verbindungen der Formel (II), Finerenone, zu entwickeln, insbesondere ein Verfahren welches sich großtechnisch gut durchführen lässt, kostengünstig ist und große Lösungsmittelüberschüsse vermeidet und mit Reagenzien auskommt die umweltfreundlicher sind.

Auch sollte der Einsatz von Aufreinigungsschritten mittels Chromatographie vermieden werden.

Mit der vorliegenden Erfindung wurde eine sehr effiziente, kürzere Synthese, ohne Verwendung von Chromatographie gefunden, die es erlaubt die oben genannten Nachteile zu umgehen. Es ist gelungen die Aminogruppe direkt in Verbindung (III) einzuführen, indem man die Verbindung (III) mit Ammoniak unter Zusatz eines Ammonium-Bromid-Salzes umsetzt.

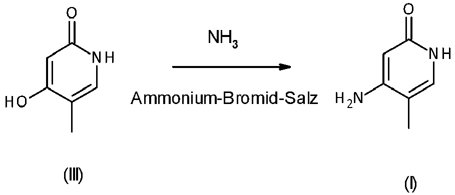

Umsetzungen von Phenolen mit Ammoniak sind in der Literatur beschrieben: In EP 2543654 A1, 2013 (Sumitomo Rubber Industries, Ltd) ist beispielsweise die Umsetzung von Phenol mit Ammoniak bei 450°C beschrieben (21,2 % Ausbeute), in I.G. Farbenind. Patent: DE570365, 1930; Fortschr. Teerfarbenfabr. Verw. Industriezweige, vol. 18, p. 446 und Fischer; Bahr; Wiedeking Brennstoff-Chemie, 1934, vol. 15, p. 101,103 sind ähnliche Verfahren unter ähnlich drastischen Bedingungen beschrieben worden. Des Weiteren in Chem. News J. Ind. Sci., 1867, vol. 16, p. 55; Helv. chim. Actc, 1924, vol. 7, p. 282; Gmelin Handbook: N: MVol.2, 5.2.2, page 490 - 493. Speziell auf die Chemie des Naphthalin bezogene Umsetzungen sind in WO2008/124812 A1, 2008 und Chemistry - An Asian Journal, 2010, vol. 5, # 9 p. 2053-2061 und US 2008/293766 A1, 2008 und EP 2075245 A2, 2009 und Synthetic Communications, 2001, vol. 31, # 14 p. 2143-2148 beschrieben. Dieses sind Sonderfälle der Bucherer Reaktion und konnten auf Verbindung (III) nicht angewendet werden, weitere Publikationen dazu: Synthetic Communications, 2001, vol. 31, # 14 p. 2143-2148; Chemische Berichte, 1924, vol. 57, p. 1738. Fortschr. Teerfarbenfabr. Verw. Industriezweige, vol. 2, p. 185; WO 2005/44786 A1, 2005; Journal für Praktische Chemie (Leipzig), 1908, vol. <2> 78, p. 153; Bulletin of the Korean Chemical Society, 2012, vol. 33, # 2 p. 387-392; Helvetica Chimica Acta, 1926, vol. 9, p. 957; Tetrahedron Letters, 2003, vol. 44, # 26 p. 4819-4822; US 4609760 A1, 1986; US 4400537 A1, 1983; Journal of the Society of Chemical Industry, London, 1932, vol. 51, p. 283. Journal of the American Chemical Society, 1944, vol. 66, p. 210, 214; US 2376112, 1942; American Chemical Journal, 1893, vol. 15, p. 40. Chemische Berichte, 1886, vol. 19, p. 1751; Journal of the American Chemical Society, 1942, vol. 64, p. 1023; Journal of Organic Chemistry, 1992, vol. 57, # 10 p. 2873-2876;

Alle in der Literatur genannten Verfahren zeichnen sich zum einen durch sehr drastische Reaktionsbedingungen wie beispielsweise Temperaturen oberhalb 300°C und teilweise niedrige Ausbeuten aus. Verfahren die mit einem Zusatz von Sulfit arbeiten oder Umsetzungen gemäß der Bucherer Reaktion sind für die Herstellung der Verbindung (I) aus (III) nicht einsetzbar. Des Weiteren sind keine Umsetzungen von hochsubstituierten "Pyridin-Phenolen" mit Ammoniak bis heute in der Literatur beschrieben.

Setzt man Verbindung (III) beispielsweise mit Ammoniakgas direkt im Autoklaven bei Temperaturen bis 180 °C um, so beobachtet man selbst nach 24 Stunden nur sehr geringen Umsatz zur Zielverbindung (I).

Es wurde überraschenderweise gefunden, dass man die Reaktion viel schonender und unter nahezu vollständigem Umsatz (in der Regel > 90% Umsatz) durchführen kann, wenn man beispielsweise 0,2 bis 3 ÄÄquivalente, bevorzugt 1 Äquivalent Ammoniumbromidsalze, wie beispielsweise Ammoniumbromid, Trimethyl-HBr, Triethylamin-HBr, Pyridin-HBr, Lutidin-HBr oder quartäre Ammoniumbromide, Tetraalkylammonium-Salze wie Tetramethylammoniumbromid, Tetrapropylammoniumbromid oder Tetrabutylammoniumbromid zur Reaktion zugibt. Bevorzugt setzt man Ammoniumbromid ein. Dann gelingt es nämlich die Reaktion direkt in reinem Ammoniak (als Reagenz und Lösungsmittel) durchzuführen. Hierzu wird Verbindung (III) und eines der oben genannten Bromide in einem Autoklaven (Hochdruck-Reaktor) vorgelegt, 20 bis 200 Äquivalente, bevorzugt 40 bis 100 Äquivalente, besonders bevorzugt 40 bis 60 Äquivalente Ammoniak einkondensiert. Die Reaktion wird bei Temperaturen von 150 bis 200°C, bevorzugt 160 bis 180°C, besonders bevorzugt bei 170°C durchgeführt, die Reaktionszeiten betragen 10 bis 40, bevorzugt 15 bis 25 Stunden. In einer besonders bevorzugten Ausführungsform wird nach beendeter Reaktion das überschüssige Ammoniak direkt in einen zweiten Autoklaven kondensiert, in dem dann die zuvor beschriebene Reaktion erneut ablaufen kann. Dieses Verfahren garantiert dann einen kontinuierlichen Betrieb, unter vollständiger Ausnutzung des Ammoniaks das heißt es werden durch den kontinuierlichen Betrieb Verluste an Ammoniak vermieden. Dieses hat aus ökologischer und ökonomischer Sicht enorme Vorteile gegen über dem Verfahren des Stands der Technik.

Der Rückstand kann nach Abdampfen des Ammoniaks in einem geeigneten Lösungsmittel aufgenommen werden und anschließend das Produkt durch Kristallisation isoliert und aufgereinigt werden. Als geeignete Lösungsmittel sind Wasser, Alkohole, wie Methanol und Ethanol, n-Butanol, sowie deren Mischungen mit Wasser geeignet. Man kann beispielsweise auch mit wenig Essigsäure lösen und das Produkt mit einem unpolaren Lösungsmittel, wie zum Beispiel Methyl-tert-butylether ausfällen. Bevorzugt aber ist eine Kristallisation aus Wasser (siehe Beispiel 1) oder im Falle von Tetraalkylammoniumbromiden das finale Ausrühren aus n-Butanol (siehe Beispiel 2). Die isolierten Ausbeuten liegen nach Trocknung in der Regel bei ca. 70 % der Theorie (eine chemische Stufe) und liegen dabei höher als die Gesamtausbeute beim Stand der Technik über zwei Stufen (62,4 % der Theorie, siehe oben). In den Mutterlaugen befinden sich in der Regel noch ca. 15-20% Produkt, das man vor allem beim Up-Scaling mitisolieren kann und damit die Gesamtausbeute signifikant > 70% der Theorie steigert. Eine chromatographische Aufreinigung, wie es im Stand der Technik beschrieben ist, ist nicht erforderlich und macht daher dieses neue erfinderische Verfahren hinsichtlich eines Up-Scalings für die Groß-Produktion sehr attraktiv.

Gegenstand der vorliegenden Erfindung ist daher ein Verfahren zur Herstellung des Verfahrensintermediats 4-Amino-5-methylpyridon der Formel (I) dadurch gekennzeichnet, dass man das Intermediat 4-hydroxy-5-methyl-1H-pyridin-2-one der Formel (III) mit Ammoniak im Autoklaven unter Zusatz eines Ammoniumbromidsalzes umsetzt.

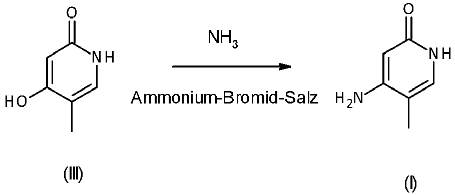

### Beispiele

### Beispiel 1

### Herstellung von 4-Amino-5-methyl-1H-pyridin-2-on (Verbindung der Formel (I)) unter Zusatz von Ammoniumbromid

4.0 g (32 mmol) 4-hydroxy-5-methyl-1H-pyridin-2-on (Verbindung III, hergestellt nach: Synthesis, S. 765 (1984)) wurden zusammen mit 3,135 g (32 mmol) Ammoniumbromid in einem Druckreaktor vorgelegt und anschließend 27,00 g (1,585 mol) Ammoniak (flüssig) ein kondensiert. Es wurde 20 Stunden bei 170°C gerührt (Druckanstieg auf 90 bar). Zur Aufarbeitung wurde das überschüssige Ammoniakgas verdampft und der Rückstand in 16 ml Wasser aufgenommen. Es wurde anschließend auf 80°C erwärmt, wobei der Rückstand in Lösung ging. Es wurde langsam abgekühlt und dann 2 Stunden bei 0-5°C nachgerührt. Die ausgefallenen Kristalle wurden abfiltriert und über Nacht im Vakuumtrockenschrank bei 50°C getrocknet.
Ausbeute: 2,70 g (68,04 % der Theorie) eines kristallinen Feststoffes.
Reinheit ca. 98 % (HPLC, 100% Methode)
MS (EIpos): m/z = 125 [M+H]+
1H-NMR (300 MHz, DMSO-d6): δ = 1.81 (s, 3H), 2.54 (s, 1H), 5.24 (s, 1H), 5.79 (s, 2H), 6.85 (s, 1H), 10.27 (br s, 1H)

### Beispiel 2

### Herstellung von 4-Amino-5-methyl-1H-pyridin-2-on (Verbindung der Formel (I)) unter Zusatz von Tetrabutylammoniumbromid

4.0 g (32 mmol) 4-hydroxy-5-methyl-1H-pyridin-2-one (Verbindung III, hergestellt nach: Synthesis, S. 765 (1984)) wurden zusammen mit 10,32 g (32 mmol) Tetrabutylammoniumbromid in einem Druckreaktor vorgelegt und anschließend 27,00 g (1,585 mol) Ammoniak (flüssig) ein kondensiert. Es wurde 20 Stunden bei 170°C gerührt (Druckanstieg auf 70 bar). Zur Aufarbeitung wurde das überschüssige Ammoniakgas verdampft und der Rückstand in 100 ml Wasser aufgenommen und mit 1N aqu. Salzsäure auf pH 7,0 gestellt und anschließend von unlöslichen Verunreinigungen abfiltriert. Es wurden 100 ml n-Butanol zugegeben und 30 Minuten bei Raumtemperatur gerührt. Die organische Phase wurde abgetrennt und nochmals mit 100 ml Wasser extrahiert. Die wässrigen Phasen wurden vereinigt und im Vakuum zur Trockene eingeengt. Der Rückstand wurde in 10 ml Wasser aufgenommen und ausgerührt. Das Produkt wurde abfiltriert und anschließend im Vakuumtockenschrank ca. 72 Stunden bei 30°C getrocknet. Das so erhaltene Produkt wurde dann final aus 10 ml n-Butanol ausgerührt, filtriert und im Vakuumtockenschrank bei 50°C getrocknet. Es wurde ein kristalliner Feststoff erhalten.
Ausbeute: 2,80 g (70,56 % der Theorie) eines kristallinen Feststoffes.
Reinheit ca. 98 % (HPLC, 100% Methode)
MS (EIpos): m/z = 125 [M+H]+ 1H-NMR (300 MHz, DMSO-d6): δ = 1.81 (s, 3H), 2.54 (s, 1H), 5.24 (s, 1H), 5.79 (s, 2H), 6.85 (s, 1H), 10.27 (br s, 1H)

**Tab. 1 Vergleich der Beispiele 1 bis 3 und des Literaturbeispiels (Synthesis, Seite 765, 1984, Beispiel 3c)**

| **Beispiel** | **Ausbeute % der Theorie** | **Synthesestufen** | **Einsatz Pd-Katalysator** | **Nebenprodukt der Formel (VI)** |
|---|---|---|---|---|
| 1 Zusatz Ammoniumbromid | 68,04 | 1 | nein | nein |
| 2 Zusatz Tetrabutylammoniumbromid | 70,56 | 1 | nein | nein |
| Literaturbeispiel aus Synthesis, Seite 765, 1984, Beispiel 3c | 62,4 | 2 | ja | ja, ca. 10 % der Gesamtausbeute |

Aus dem oben beschreiben wird deutlich, dass an den bisherigen zur Verfügung stehenden Verfahren, wie es in Synthesis, Seite 765, 1984, Beispiel 3c beschreiben ist, nachteilig sind, dass
(1) eine mehrstufige Synthese durchgeführt wird,
(2) das Nebenprodukt der Formel (VI) anfällt (bis > 10%), welches als Verunreinigung bei der Verbindung der Formel (I) auftritt und durch aufwendige Chromatographische Verfahren abgetrennt werden muss,
(3) Benzylamin in einem sehr großen Überschuss verwendet wird, dessen Recycling aufwendig und mit erheblichen Kosten verbunden ist,
(4) die Reaktion in siedendem Benzylamin bei 185°C und mit einer Reaktionszeit von 36 Stunden durchgeführt werden muss da solche hohen Temperaturen in Standard-Rührwerken nicht durchführbar sind und erfordern spezielles technisches Equipment erfordert,
(5) chlorierte Lösungsmittel eingesetzt werden, die nicht umweltfreundlich sind, und
(6) große Mengen Pd-Katalysator auf Kohle eingesetzt werden müssen, dessen Abtrennung und Aufarbeitung nicht nur aufwendig, sondern auch in der großtechnischen Synthese kaum realisierbar sind.

Im Gegensatz dazu werden diese Nachteile durch das erfindungsgemäße Verfahren vermieden und die folgenden Effekte und Vorteile erzielt:
(1) es wird nur eine Verfahrensstufe durchgeführt,
(2) die isolierten Ausbeuten der Verbindung nach Formel (III) liegen nach Trocknung in der Regel bei ca. 70 % der Theorie (eine chemische Stufe) und liegen dabei höher als die Gesamtausbeute beim Stand der Technik über zwei Stufen (62,4 % der Theorie, Synthesis, Seite 765, 1984, Beispiel 3c),
(3) in der Mutterlauge, die in dem erfindungsgemäßen Verfahren anfällt, befindet sich in der Regel noch ca. 15-20% Produkt, das man vor allem beim Up-Scaling mitisolieren kann, wodurch die Gesamtausbeute signifikant > 70% der Theorie gesteigert werden kann,
(4) die Verbindung nach der Formel (I) wird direkt, ohne Aufreinigung, in hoher Reinheit als kristallines Produkt gewonnen,
(5) der Einsatz Pd-Katalysator auf Kohle wird vermieden,
(6) die Verbindung der Formel (VI) fällt nicht als unerwünschtes Nebenprodukt an,
(7) chromatographische Aufreinigung, wie es im Stand der Technik beschrieben ist, ist nicht erforderlich und macht daher dieses neue erfinderische Verfahren hinsichtlich eines Up-Scalings für die Groß-Produktion sehr attraktiv
(8) eine chromatographische Aufreinigung, wie es im Stand der Technik beschrieben ist, ist nicht erforderlich, da das unerwünschte Nebenprodukt der Formel (VI) nicht anfällt,
(9) es kann auf den mehrfachen Einsatz von Lösungsmitteln, insbesondere chlorierten Lösungsmitteln, ganz oder teilweise verzichtet werden, so dass das erfindungsgemäße Verfahren deutlich umweltfreundlicher ist und
(10) deutlich geringere Reaktionszeiten und/oder Reaktionstemperaturen erfordert.

Insgesamt stellt das erfindungsgemäße Verfahren eine sehr effiziente, kürzere Synthese, ohne Verwendung von Chromatographie dar, dass auch für ein Up-Scaling geeignet ist. Es ist gelungen die Aminogruppe direkt in Verbindung der Formel (III) einzuführen, indem man die Verbindung der Formel (III) mit Ammoniak unter Zusatz eines Ammonium-Bromid-Salzes umsetzt.

Die folgenden Absätze beschreiben Ausführungsformen der Erfindung:
(1.) Verfahren zur Herstellung des Verfahrensintermediats 4-Amino-5-methylpyridon der Formel (I) dadurch gekennzeichnet, dass man das Intermediat 4-hydroxy-5-methyl-1H-pyridin-2-one der Formel (III) mit Ammoniak im Autoklaven unter Zusatz eines Ammoniumbromidsalzes umsetzt. 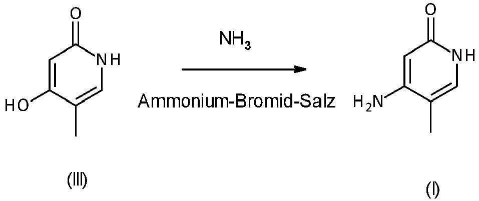
(2.) Verfahren gemäß Absatz 1, dadurch gekennzeichnet, dass man die Reaktion bei 150 bis 200°C durchführt.
(3.) Verfahren nach einem der Absätze 1 oder 2, dadurch gekennzeichnet, dass man Ammoniumbromid einsetzt.
(4.) Verfahren nach einem der Absätze 1 bis 3, dadurch gekennzeichnet, dass man 1 Äquivalent Ammoniumbromidsalz einsetzt.
(5.) Verfahren nach einem der Ansprüche 1 bis 4 dadurch gekennzeichnet, dass man 20 bis 200 Äquivalente Ammoniak einsetzt.
(6.) Verfahren nach einem der Absätze 1 bis 5, dadurch gekennzeichnet, dass man nach Abschluss der Reaktion das überschüssige Ammoniak direkt in einen zweiten Autoklaven kondensiert in dem dann die Reaktion gemäß der Absätze 1 bis 5 durchgeführt wird.

## Patentansprüche

1. Verfahren zur Herstellung des Verfahrensintermediats 4-Amino-5-methylpyridon der Formel (1) 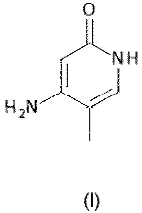 wobei das Intermediat 4-Hydroxy-5-rnethyl-1H-pyridin-2-on der Formel (III) 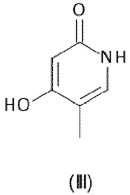 mit Ammoniak im Autoklaven unter Zusatz eines Ammoniumbromidsalzes umgesetzt wird.

2. Verfahren nach Anspruch 1, wobei die Reaktion bei 150 bis 200°C durchgeführt wird.

3. Verfahren nach Anspruch 1 oder 2, wobei die Reaktion bei 160 bis 180°C durchgeführt wird.

4. Verfahren nach einem der Ansprüche 1 bis 3, wobei die Reaktion bei 170°C durchgeführt wird.

5. Verfahren nach einem der Ansprüche 1 bis 4, wobei das Ammoniumsalz ausgewählt ist, aus der Gruppe, bestehend aus Ammoniumbromid, Trimethyl-HBr, Triethylamin-HBr, Pyridin-HBr, Lutidin-HBr, quartäre Ammoniumbromide, Tetraalkylammonium-Salze, Tetramethylammoniumbromid, Tetrapropylammoniumbromid und Tetrabutylammoniumbromid.

6. Verfahren nach Anspruch 5, wobei das Ammoniumsalz Ammoniumbromid ist.

7. Verfahren nach einem der Ansprüche 1 bis 6, wobei 0,2 bis 3 Äquivalente des Ammoniumbromidsalzes eingesetzt werden.

8. Verfahren nach einem der Ansprüche 1 bis 7, wobei 1 Äquivalent des Ammoniumbromidsalzes eingesetzt wird.

9. Verfahren nach einem der Ansprüche 1 bis 8, wobei 20 bis 200 Äquivalente Ammoniak eingesetzt werden.

10. Verfahren nach einem der Ansprüche 1 bis 9, wobei 40 bis 100 Äquivalente Ammoniak eingesetzt werden.

11. Verfahren nach einem der Ansprüche 1 bis 10, wobei 40 bis 60 Äquivalente Ammoniak eingesetzt werden.

12. Verfahren nach einem der Ansprüche 1 bis 11, wobei nach Abschluss der Reaktion das überschüssige Ammoniak direkt in einen zweiten Autoklaven kondensiert wird, in dem dann die Reaktion gemäß der Ansprüche 1 bis 11 durchgeführt wird.

## Claims

1. Method for preparing the process intermediate 4-amino-5-methylpyridone of the formula (I) 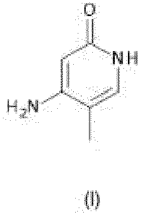 wherein the intermediate 4-hydroxy-5-methyl-1H-pyridin-2-one of the formula (III) 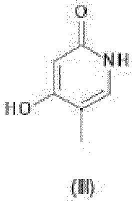 is reacted with ammonia in an autoclave with addition of an ammonium bromide salt.

2. Method according to Claim 1, wherein the reaction is carried out at 150 to 200°C.

3. Method according to Claim 1 or 2, wherein the reaction is carried out at 160 to 180°C.

4. Method according to any of Claims 1 to 3, wherein the reaction is carried out at 170°C.

5. Method according to any of Claims 1 to 4, wherein the ammonium salt is selected from the group consisting of ammonium bromide, trimethyl-HBr, triethylamine-HBr, pyridine-HBr, lutidine-HBr, quaternary ammonium bromides, tetraalkylammonium salts, tetramethylammonium bromide, tetrapropylammonium bromide and tetrabutylammonium bromide.

6. Method according to Claim 5, wherein the ammonium salt is ammonium bromide.

7. Method according to any of Claims 1 to 6, wherein 0.2 to 3 equivalents of the ammonium bromide salt are used.

8. Method according to any of Claims 1 to 7, wherein 1 equivalent of the ammonium bromide salt is used.

9. Method according to any of Claims 1 to 8, wherein 20 to 200 equivalents of ammonia are used.

10. Method according to any of Claims 1 to 9, wherein 40 to 100 equivalents of ammonia are used.

11. Method according to any of Claims 1 to 10, wherein 40 to 60 equivalents of ammonia are used.

12. Method according to any of Claims 1 to 11, wherein, after the reaction has ended, the excess ammonia is condensed directly into a second autoclave in which the reaction according to Claims 1 to 11 is then carried out.

## Revendications

1. Procédé de préparation de l'intermédiaire de procédé 4-amino-5-méthylpyridone de formule (I) 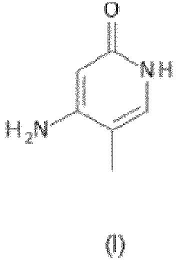 , l'intermédiaire 4-hydroxy-5-méthyl-1H-pyridin-2-one de formule (III) 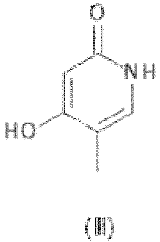 étant transformé avec de l'ammoniac dans un autoclave avec ajout d'un sel de bromure d'ammonium.

2. Procédé selon la revendication 1, la réaction étant mise en oeuvre à une température de 150 à 200 °C.

3. Procédé selon la revendication 1 ou 2, la réaction étant mise en oeuvre à une température de 160 à 180 °C.

4. Procédé selon l'une quelconque des revendications 1 à 3, la réaction étant mise en oeuvre à une température de 170 °C.

5. Procédé selon l'une quelconque des revendications 1 à 4, le sel d'ammonium étant choisi dans le groupe constitué par le bromure d'ammonium, le triméthyl-HBr, la triéthylamine-HBr, la pyridine-HBr, la lutidine-HBr, des bromures d'ammonium quaternaires, des sels de tétraalkylammonium, le bromure de tétraméthylammonium, le bromure de tétrapropylammonium et le bromure de tétrabutylammonium.

6. Procédé selon la revendication 5, le sel d'ammonium étant le bromure d'ammonium.

7. Procédé selon l'une quelconque des revendications 1 à 6, 0,2 à 3 équivalents du sel de bromure d'ammonium étant utilisés.

8. Procédé selon l'une quelconque des revendications 1 à 7, 1 équivalent du sel de bromure d'ammonium étant utilisé.

9. Procédé selon l'une quelconque des revendications 1 à 8, 20 à 200 équivalents d'ammoniac étant utilisés.

10. Procédé selon l'une quelconque des revendications 1 à 9, 40 à 100 équivalents d'ammoniac étant utilisés.

11. Procédé selon l'une quelconque des revendications 1 à 10, 40 à 60 équivalents d'ammoniac étant utilisés.

12. Procédé selon l'une quelconque des revendications 1 à 11, dans lequel après la fin de la réaction, l'ammoniac en excès est directement condensé dans un deuxième autoclave, dans lequel la réaction selon les revendications 1 à 11 est alors mise en oeuvre.
